(19) **Europäisches Patentamt**

**European Patent Office**

**Office européen des brevets**

(11) **EP 1 069 434 A2**

(12) **EUROPÄISCHE PATENTANMELDUNG**

(43) Veröffentlichungstag:
17.01.2001 Patentblatt 2001/03

(51) Int. Cl.[7]: **G01N 33/72**

(21) Anmeldenummer: **00114153.0**

(22) Anmeldetag: **12.07.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **14.07.1999 DE 19933275**

(71) Anmelder:
**Fresenius Kabi Deutschland GmbH**
**61159 Friedberg (DE)**

(72) Erfinder:
• **Eichner, Wolfram, Dr.**
**35510 Butzbach (DE)**
• **Bepperling, Frank, Dr.**
**61231 Bad Nauheim (DE)**
• **Sommermeyer, Klaus, Dr.**
**61191 Rosbach v.d.H. (DE)**
• **Opitz, Jens**
**60437 Frankfurt (DE)**

(74) Vertreter: **UEXKÜLL & STOLBERG**
**Patentanwälte**
**Beselerstrasse 4**
**22607 Hamburg (DE)**

(54) **Verwendung von Hämoglobin-Derivativen zur Bestimmung des Plasma- und/oder Blutvolumens**

(57) Die Erfindung betrifft die Verwendung von Hämoglobin-Derivaten zur Bestimmung des Plasma- und/oder Blutvolumens, wobei es sich bei den Hämoglobin-Derivaten um stabile Chromoproteine mit einem Molekulargewicht von mindestens 64 kDa handelt. Die Verwendung kann eine Behandlung eines Patienten umfassen, bei der man

(a) einem Patienten eine bestimmte Menge des Hämoglobin-Derivates appliziert,

(b) die Vermischung des Hämoglobin-Derivates mit dem Blut des Patienten abwartet,

(c) dem Patienten mindestens eine Probe entnimmt,

(d) den Gehalt des Hämoglobin-Derivates und/oder den Gehalt des von den Hämoglobin-Derivaten gebundenen Liganden in der Probe bestimmt und

(e) das Plasma- und/oder Blutvolumen daraus ermittelt.

Die Erfindung betrifft auch Mittel zur Bestimmung des Plasma- und/oder Blutvolumens, die Hämoglobin-Derivate enthalen und dadurch gekannzeichnet sind, daß es sich bei den Hämoglobin-Derivaten um stabile Chromoproteine mit einem Molekulargewicht von mindestens 64 kDa handelt, die mindestens teilweise Kohlenmonoxid als Liganden tragen, sowie Verfahren zu deren Herstellung.

EP 1 069 434 A2

**Beschreibung**

Die vorliegende Erfindung betrifft die Verwendung von Hämoglobin-Derivaten zur Bestimmung des Plasma- und/oder Blutvolumens sowie Mittel zur Bestimmung des Plasma- und/oder Blutvolumens, die ein Hämoglobin-Derivat mit Kohlenmonoxid als Liganden enthalten.

Als Blutvolumen wird die Gesamtmenge des zirkulierenden Blutes bezeichnet, welche sich aus dem Plasmavolumen und dem Erythrozytenvolumen zusammensetzt. Zur Aufrechterhaltung aller Organfunktionen ist eine ausreichende Durchblutung der Organe notwendig. Blut- und Flüssigkeitsverluste führen zu einer Umverteilung des Blutvolumens, um die lebenswichtigen Funktionen zu erhalten. Dabei kann es zur verringerten Durchblutung einiger Organe und als Folge dessen zu einer Schädigung derselben kommen. Das Plasmavolumen als Maß für den Füllungszustand des kardiovaskulären Systems und zur Bestimmung der ausreichenden Organdurchblutung bei Patienten mit Blut- oder Flüssigkeitsverlusten ist somit für den klinischen Alltag und für die Forschung von großer Bedeutung.

Im Stand der Technik wurden daher eine Vielzahl von Verfahren entwickelt, mit denen das Plasma- und/oder Blutvolumen bestimmt werden können.

Standardverfahren zur Bestimmung des Volumens der Erythrozyten und des Plasmavolumens unter Verwendung von radioaktiven Markierungen wurden bereits 1973 von der Internationalen Gesellschaft für Hämatologie vorgeschlagen (British Journal of Hematology, Vol. 25 (1973), 801-814). Zur Bestimmung des Volumens der Erythrozyten wurde ein Verfahren empfohlen, bei dem man Erythrozyten eines Patienten mit einer Lösung vermischt, die $^{51}$Cr enthält, die Mischung für 15 Minuten inkubiert und die Erythrozyten anschließend gewinnt und wäscht. Die radioaktiv markierten Erythrozyten werden dem Patienten wieder injiziert. 10 und 20 Minuten nach der Injektion werden dem Patienten Blutproben von 5 bis 10 ml entnommen, in denen die Erythrozyten lysiert werden und das Volumen anhand der gemessenen Radioaktivität bestimmt wird.

Bei dem entsprechenden Standardverfahren zur Bestimmung des Plasmavolumens gibt man zu dem Plasma des Patienten mit radioaktivem Jod markiertes humanes Serumalbumin. Die Probennahme und Auswertung erfolgt im wesentlichen wie bei dem Verfahren zur Bestimmung des Volumens der Erythrozyten.

Aufgrund der Strahlenbelastung ist eine routinemäßige Injektion, insbesondere eine wiederholte Gabe, von radioaktiv-markierten Isotopen im klinischen Alltag völlig ausgeschlossen. Dementsprechend werden diese Standardverfahren nur noch für wissenschaftliche Zwecke oder bei speziellen Krankheitsbildern, wie beispielsweise Polyzythämia vera, angewendet.

Ferner wurden im Stand der Technik Verfahren zur Bestimmung des Plasmavolumens entwickelt, bei denen Farbstoffkomplexe dem Blut der Patienten als Analyten zugegeben werden. Dabei injiziert man beispielsweise Evans Blue oder Indocyaningrün (ICG) intravenös und bestimmt die Konzentration der Farbstoffe spektrophotometrisch oder densitometrisch nach Durchmischung mit dem Blutplasma und Entnahme einer Probe (Haller et al., Anästhesist, Vol. 41 (1992), 115-120; und Gehring et al., Infusionstherapie und Transfusionsmedizin, Vol. 23 (1996), 86-91). Aus der Verdünnung des Farbstoffs durch das Plasma kann das Plasmavolumen ermittelt werden. Es hat sich jedoch herausgestellt, daß auch diese Verfahren Risiken für die Patienten enthalten. Der Farbstoff Evans Blue steht im Verdacht, mutagene Eigenschaften zu besitzen, und ist daher für die Bestimmung des Plasmavolumens beim Menschen ungeeignet. Die Zugabe von ICG soll zentralvenös oder zentralarteriell erfolgen und die Proben sollen arteriell gewonnen werden. Dem Patienten müssen daher zentralvenöse und/oder arterielle Kathether gelegt werden, um die Messung durchzuführen. Das Legen bzw. Einschwemmen der Katheter ist nicht ohne Gefahren für den Patienten und sehr aufwendig. Ferner interferiert ICG, das an Plasmaprotein gebunden ist, mit der Pulsoximetrie, einem klinisch routinemäßig angewendeten, nicht invasiven Verfahren zur Bestimmung der Sauerstoffsättigung (Scheuer et al., Anesthesiology, Vol. 65 (1986), 550-552) und besitzt lediglich eine Plasmhalbwertszeit von 3,2 min. Der Farbstoff wird ausschließlich hepatisch eliminiert (Haller et al., a.a.O.). Bei Plasmaproteinverlusten, beispielsweise bei der Extravasion von Albumin aufgrund von Verbrennungen, werden bei der Bestimmung des Plasmavolumens aufgrund der Bindung des ICG an Plasmaproteine zu hohe Plasmavolumina ermittelt.

Obwohl die Plasmavolumenbestimmung mit ICG bereits seit über 30 Jahren im Stand der Technik bekannt ist (Bradley, E.C. und Barr, J.W., Life Sci., Vol. 7 (1968), 1001-1007), konnte sich dieses Verfahren aufgrund der genannten Probleme im klinischen Alltag nicht durchsetzen.

Auch die Bestimmung des Volumens der Erythrozyten durch Markierung mit dem Farbstoff Fluorescein wurde bereits im Stand der Technik durchgeführt (Ohrt et al., Ansth. Analg., Vol. 87 (1998), 1234-1238). Dabei markiert man die Erythrozyten eines Patienten mit Fluorescein und re-injiziert diese dem Patienten. Nach Durchmischung des Blutes werden Proben entnommen und mittels Durchflußzytometrie auf den Gehalt an Fluorescein hin analysiert. Aufgrund der Tatsache, daß die Markierung der Erythrozyten und die nachfolgende Analyse mehr eine Stunde dauert, ist die Anwendbarkeit dieses Verfahrens auf Problemstellungen beschränkt, in denen die akute Bestimmung des Volumenstatus von geringer Relevanz ist.

Schließlich wurden Verfahren zur Bestimmung des Blutvolumens entwickelt, bei denen man einem Patienten eine definierte Menge an Kohlenmonoxid mit dem Atemgas zuführt und anschließend die Kohlenmonoxid-Sättigung des Blutes bestimmt (Christensen et al., Anaesthesiol. Scand., Vol. 730 (1993), 622-627; und Poulsen et al., Eur. J. Appl. Physiol, Vol. 77 (1998), 457-461). Die Applikation einer definierten Kohlenmonoxid-Menge ist sehr aufwendig und auf Patienten beschränkt, die künstlich beatmet werden.

Gemäß einer weiteren Ausführungsform dieses Verfahrens entnimmt man Patienten Blut und behandelt es mit Kohlenmonoxid. Die Erythrozyten, die mit Kohlenmonoxid beladenes Hämoglobin enthalten, werden re-injiziert und die Konzentration des Kohlenmonoxids im Blut wird nach Durchmischung und Probennahme bestimmt (Obata et al., British Journal of Anaesthesia, Vol. 81 (1998), 940-944). Dieses Verfahren verringert jedoch die Sauerstofftransportkapazität des Blutes, da ein Teil des Blutes der Patienten durch das gebundene Kohlenmonoxid an der Aufnahme von Sauerstoff gehindert wird. Diese Verfahren können daher bei Patienten mit niedriger Sauerstoffreserve nicht angewendet werden.

Aufgrund der im Stand der Technik beschriebenen Probleme mit Verfahren zur unmittelbaren Bestimmung des Plasma- und/oder Blutvolumens werden diese im klinischen Alltag nicht routinemäßig angewendet. Der Volumenstatus der Patienten wird daher nach wie vor durch Messung hämodynamischer Kreislaufparameter wie Blutdruck, Herzfrequenz, pulmonale Drücke und Herzzeitvolumen indirekt ermittelt. Diese Messungen ermöglichen jedoch keine Aussagen über das totale und periphere Blut- bzw. Plasmavolumen.

Aufgabe der vorliegenden Erfindung ist es somit, ein Mittel zur Verfügung zu stellen, welches zur Bestimmung des Plasma- und Blutvolumens im klinischen Alltag verwendbar ist.

Diese Aufgabe wird erfindungsgemäß durch die Verwendung von Hämoglobin-Derivaten zur Bestimmung des Plasma- und/oder Blutvolumens gelöst. Bei den Hämoglobin-Derivaten handelt es sich um stabile Chromoproteine mit einem Molekulargewicht von mindestens 64 kDa.

Im Rahmen der vorliegenden Erfindung konnte überraschenderweise gezeigt werden, daß diese Hämoglobin-Derivate in besonderem Maße zur Bestimmung des Plasmavolumens geeignet sind, da die Hämoglobin-Derivate und/oder an diese gebundene Liganden in geringer Konzentration nachweisbar sind. Dadurch wird die Applikation der Hämoglobin-Derivate in einer Menge möglich, die weder eine Veränderung des Volumens noch eine Änderung anderer Parameter des Blutes herbeiführt.

Die vorliegende Erfindung betrifft ferner die Verwendung der Hämoglobin-Derivate in einem diagnostischen Verfahren zur Bestimmung des Plasma- und/oder Blutvolumens, bei dem man einem Patienten eine bestimmte Menge des entsprechenden Hämoglobin-Derivates appliziert, die Vermischung des Hämoglobin-Derivates mit dem Blut des Patienten abwartet, mindestens eine Probe von dem Patienten entnimmt, den Gehalt des Hämoglobin-Derivates oder den Gehalt des von den Hämoglobin-Derivaten gebundenen Liganden in der Probe bestimmt und das Plasma- und/oder Blutvolumen ermittelt.

Die Verdünnung des applizierten Hämoglobin-Derivates kann dabei entweder durch Bestimmung der Hämoglobin-Konzentration im Blut oder Plasma und/oder durch Bestimmung der Konzentration des von dem Hämoglobin-Derivat gebundenen Liganden erfolgen.

Weitere Gegenstände der vorliegenden Erfindung sind Mittel zur Bestimmung des Plasma- und/oder Blutvolumens, die Hämoglobin-Derivate enthalten, an welche mindestens teilweise als Liganden Kohlenmonoxid gebunden ist. Gemäß einer bevorzugten Ausführungsform sind die Hämoglobin-Derivate vollständig mit Kohlenmonoxid gesättigt.

Im Rahmen der vorliegenden Erfindung wird durch die Verwendung der Hämoglobin-Derivate somit erstmals die Möglichkeit zur Verfügung gestellt, das Plasma- und/oder Blutvolumen mittels eines einfachen, schnellen und sicheren Verfahrens zu bestimmen, das alle Voraussetzungen für die Anwendung im klinischen Alltag erfüllt.

Hämoglobin ist ein Chromoprotein mit einem Molekulargewicht von 64 Kilodalton (kDa). Das Protein besteht aus zwei $\alpha$- und $\beta$-Globinketten, die als prosthetische Gruppe jeweils ein Häm gebunden haben. Isolierte Hämoglobin-Moleküle sind instabil und zerfallen schnell in die stabileren $\alpha$-/$\beta$-Dimere mit einem Molekulargewicht von 32 kDa.

Im Rahmen der vorliegenden Erfindung werden als "Hämoglobin-Derivate" Moleküle bezeichnet, die $\alpha$- und/oder $\beta$-Globinketten enthalten, welche intramolekular vernetzt wurden, um ein stabiles Chromoprotein mit einem Molekulargewicht von mindestens 64 kDa zu erhalten. Vorzugsweise bestehen die Hämoglobin-Derivate aus zwei $\alpha$- und zwei $\beta$-Globinketten.

Die Vernetzung kann beispielsweise durch kovalente Bindung der Globinketten sowie durch die Fusion der Gene, die für die Globinketten kodieren, erfolgen. Derartige Kopplungsprodukte bestehend aus $\alpha$-/$\alpha$-Hämoglobinketten, die mittels eines "Verbindungsstückes" verknüpft sind, wurden bereits im Stand der Technik beschrieben (EP 402 300, EP 700 997 US 5,844,090 oder Kerwin et al., J. of Pharmaceutical Science, Vol. 88:1, (1999) 79).

Selbstverständlich umfaßt der Begriff Hämoglobin-Derivate auch Hämoglobin-Moleküle, deren Primärstruktur durch Substitution, Deletion oder Addition von Aminosäuren verändert wurde, sofern die erfindungswesentlichen Eigenschaften des Derivates (Chromoprotein mit einem Molekulargewicht von mindestens 64 kDa) durch diese Änderungen nicht wesentlich beeinträchtigt werden. Entsprechende allelische Varianten des Hämoglobins kommen natürlich vor oder können rekombinant erzeugt werden. Kerwin et al. (a.a.O.) verwenden beispielsweise eine bekannte Mutation des Hämoglobins, um ein Hämoglobin-Derivat mit reduzierter Sauerstoff-Affinität zu erzeugen. WO 98/50430 beschreibt Änderungen an der Primärstruktur des Hämoglobins, die eine verbesserte Löslichkeit oder eine reduzierte Stickstoff-Bindung des Moleküls bewirken.

Der Begriff Hämoglobin-Derivate umfaßt ferner Hämoglobin-Moleküle, die zur Bildung von polymeren Hämoglobin-Formen intermolekular verknüpft, an Liganden, wie beispielsweise Polyethylenglycol (PEG; vgl. US 5,478,806) oder Hydroxyethylstärke (HES; vgl. WO 98/01158) und/oder an andere Polymere gekoppelt wurden. Die intramolekulare Vernetzung kann vor, gleichzeitig mit oder nach der intermolekularen Verknüpfung erfolgen. Bei der Bindung an einen Liganden erfolgt die intramolekulare Vernetzung durch die Reaktion mit der die Globinketten mit dem Liganden verbunden werden.

Das Hämoglobin, das für die Herstellung der Derivate verwendet wird, kann menschlichen, tierischen oder rekombinanten Ursprungs sein. In der Literatur wurden verschiedene Verfahren zur Herstellung von rekombinantem Hämoglobin beschrieben (Expression in Bakterien-, Hefe- oder tierischen Zellen, sowie in transgenen Pflanzen oder Tieren).

Die Herstellung solcher Hämoglobin-Derivate zur Gewinnung von "Hemoglobin based oxygen carriers" (HBOCs) ist im Stand der Technik umfassend beschrieben (Benesch, Meth. Enzymol., Vol. 231 (1994), 267-274; Keipert et al., Transfusion, Vol. 29 (1989), 767-773; Snyder et al., Proc. Natl. Acad. Sci. USA, Vol. 84 (1987), 7280-7284; Rogers et al., Biochim. et Biophys. Acta, Vol. 1248 (1995), 135-142; Hai et al., Art. Cells, Blood Subs. and Immob. Biotech, Vol. 22(3) (1994), 923-931; DE 26 07 706, DE 26 16 086; EP 646 130; EP 290 252; EP 277 289; WO 98/0115; U.S. 4,911,929; U.S. 4,861,867; U.S. 4,857,636; U.S. 4,777,244; U.S. 4,698,387; U.S. 4,600,531; U.S. 4,526,715; U.S. 4,473,494; und U.S. 4,301,144). Für die vorliegende Erfindung können beliebige Hämoglobin-Derivate verwendet werden, wobei es im Rahmen der vorliegenden Erfindung nicht erforderlich ist, daß diese Derivate Sauerstoff reversibel binden können.

Das intramolekular vernetzte Hämoglobin weist ein Molekulargewicht von mindestens 64 kDa auf, wobei vernetzte Hämoglobin-Derivate oder -Konjugate ab einem Molekulargewicht von mindestens 128 kDa besonders bevorzugt sind. Moleküle dieser Größe haben den besonderen Vorteil, daß sie den Intravasalraum nur in sehr geringem Umfang verlassen können. Die Ermittlung eines zu hohen Plasma- und/oder Blutvolumens durch Extravasion des Analyten wird dadurch ausgeschlossen. Vorzugsweise sollten die Hämoglobin-Derivate für die erfindungsgemäße Verwendung eine Obergrenze von 700 kDa nicht überschreiten, wobei Molekulargewicht von bis zu 500 kDa besonders bevorzugt ist.

Das Verfahren zur Herstellung des erfindungsgemäßen Mittels zur Bestimmung des Plasma- und/oder Blutvolumens kann die Vermischung des Hämoglobin-Derivates mit einer physiologischen Lösung, gegebenenfalls zusammen mit einem geeigneten Hilfs- oder Trägerstoff, umfassen. Dabei können beliebige im Stand der Technik bekannte Träger- und/oder Hilfsstoffe verwendet werden (z.B. Ringer Laktat, Natriumchlorid, Natriumphosphat, Polysorbate und/oder EDTA enthaltende Lösungen; für einen allgemeinen Überblick vgl. Kerwin et al., a.a.O.).

Im Stand der Technik sind eine Vielzahl von Bedingungen (z.B. Temperatur und Zeit) beschrieben, bei denen Hämoglobin-Derivate in Abhängigkeit von dem Liganden stabil gelagert werden können. Diese Bedingungen können im Rahmen der vorliegenden Erfindung zum Einsatz kommen. Um eine Oxidation des Hämoglobins zu Met-Hämoglobin bei der Lagerung zu verhindern, kann beispielsweise ein Reduktionsmittel oder ein Stabilisator zugegeben werden. Die Verwendung von Ascorbinsäure, N-Acetyltryptophan oder N-Acetylcystein als Stabilisator ist bevorzugt.

Die Verpackung der Hämoglobin-Derivate kann in beliebige im Stand der Technik beschriebene Behälter erfolgen. Gemäß einer Ausführungsform der Erfindung werden Glas- oder Kunststoff-Behälter zur Verpackung verwendet, wobei die Verwendung von Sauerstoff-undurchlässigen Behältern für Hämoglobin-Derivate bevorzugt sind. Gemäß einer besonders bevorzugten Ausführungsform der Erfindung werden Hämoglobin-Derivate in der Oxy- oder Desoxy-Form, also ohne Liganden oder mit Sauerstoff als Liganden, in Sauerstoff-undurchlässigen Behältern gelagert.

Zur Verpackung können ferner Folien aus Ethyl-Vinyl Acetat (EVA), Polyethylen (PE), Polyamid (PA), Polyolefinen, Polyvinylchlorid (PVC) oder Verbundfolien, z.B. aus PE/PA eingesetzt werden. Eine weitere Möglichkeit der Verpackung besteht darin, die Hämoglobin-Derivate in Fertigspritzen einzubringen.

Verfahren zur weiteren Reduzierung des Sauerstoff-Gehaltes während oder nach der Verpackung sind ebenfalls im Stand der Technik bekannt (vgl. Kerwin et al, 1999, a.a.O.) und können im Rahmen der vorliegenden Erfindung zum Einsatz kommen. Sauerstoff-durchlässige Verpackungsmaterialen können zusätzlich mit einem Sauerstoff-undurchlässigen Umbeutel verpackt werden, der gegebenenfalls Sauerstoffabsorber, wie z.B. Ageless, enthalten kann.

Die erfindungsgemäße Verwendung von Hämoglobin-Derivaten zur Bestimmung des Plasma- und/oder Blutvolumens kann Schritte umfassen, bei denen man einem Patienten eine bestimmte Menge des Hämoglobin-Derivates appliziert, die Vermischung des Hämoglobin-Derivates mit dem Blut des Patienten abwartet, mindestens eine Probe von dem Patienten entnimmt, den Gehalt des Hämoglobin-Derivates oder den Gehalt des von den Hämoglobin-Derivaten gebundenen Liganden in der Probe bestimmt und das Plasma- und/oder Blutvolumen ermittelt.

Gemäß eines weiteren Aspektes ermöglicht die erfindungsgemäße Verwendung der Hämoglobin-Derivate zur Bestimmung des Plasma- und/oder Blutvolumens es, Patienten mit Blutverlust ein Hämoglobin-Derivat zu geben, das gleichzeitig als Sauerstoff-Träger wirkt und für die Bestimmung des Plasma- und/oder Blutvolumens verwendet werden kann.

Die erfindungsgemäßen Verwendung von Hämoglobin-Derivaten in diagnostischen Verfahren zur Bestimmung des Plasma- und/oder Blutvolumens kann sowohl bei Menschen, wie auch bei Tieren durchgeführt werden.

Gemäß einer bevorzugten Ausführungsform der Erfindung erfolgt die Applikation des Hämoglobin-Derivates durch zentral oder peripher venöse Injektion. Grundsätzlich sollte die Menge des zu applizierenden Hämoglobin-Derivates so gering wie möglich gehalten werden, wobei die Untergrenze durch die Nachweisgrenze des Derivates in der folgenden Analyse bestimmt ist. Die Nachweisgrenze selbst hängt im Einzelfall wiederum von dem verwendeten Analyseverfahren ab.

Die jeweils zu applizierende Menge der Hämoglobin-Derivate kann somit vom Fachmann in Abhängigkeit der genannten Parameter im Einzelfall leicht bestimmt werden. Vorzugsweise werden 0,05 bis 0,3 g/kg Körpergewicht des Hämoglobin-Derivates appliziert.

Nach Applikation des Hämoglobin-Derivates vermischt sich dieses innerhalb weniger Minuten völlig mit dem Blut des Patienten. Die Verteilungsgeschwindigkeit hängt dabei von der Art der Applikation und von der klinischen Situation des Patienten ab.

Anschließend wird mindestens eine Probe des Blutes des Patienten entnommen, wobei die Entnahme von zwei bis drei Proben bevorzugt ist. Die Proben können dabei beispielsweise intraarteriell oder intravenös entnommen werden.

Das Volumen der Probe wird so gewählt, daß eine Analyse der Konzentration des Hämoglobin-Derivates und/oder des von diesem Derivat gebundenen Liganden in der Probe möglich ist. Gemäß einer bevorzugten Ausführungsform der Erfindung werden maximal 2 bis 3 ml Blut entnommen.

Die Bestimmung der Hämoglobin-Konzentration in der Probe kann nach im Stand der Technik bekannten Verfahren erfolgen. Beispielsweise kann das Verfahren nach Drabkins zum Einsatz gelangen (International Committee for Standardization in Haematology in J. Clin. Path., Vol. 18 (1965), 71-75; and Br. J. Haematol., Vol. 13 (1997), 71-75). Alternativ dazu kann die Hämoglobin-Konzentration mittels des aca® DuPont Discrete Clinical Analyzer bestimmt werden, welcher die Bestimmung nach einer Abwandlung des Drabkins-Verfahrens durchführt (vgl. Anweisungen des Herstellers, "Methode 91"). Beide Verfahren verwenden ein optisches Meßprinzip. Schließlich kann die Hämoglobin-Konzentration auch mittels des hämolytischen Index auf einem Hitachi-Analysegerät bestimmt werden.

Aus der gemessenen Absorption oder Extinktion des Hämoglobin-Derivates kann man die Konzentration des Analyten über eine Eichkurve bestimmen. Die Eichkurve kann beispielsweise erstellt werden, indem die Absorption oder Extinktion von mindestens zwei bekannten Konzentrationen der Hämoglobin-Derivate ermittelt wird. Die Erstellung einer Eichkurve aus mindestens vier bekannten Konzentrationen der Hämoglobin-Derivate ist jedoch bevorzugt.

Für die Bestimmung des Plasma- und/oder Blutvolumens kann es vorteilhaft sein, eine Probe von dem Patienten vor der Zugabe des Hämoglobin-Derivates zu entnehmen, um die Konzentration an Plasma-Hämoglobin vor Injektion des Derivates zu bestimmen (Ermittlung des "Null-Wertes").

Gemäß einer besonders bevorzugten Ausführungsform der Erfindung werden für die Bestimmung des Plasma- und/oder Blutvolumens mehrere Proben von dem Patienten nach Injektion des Hämoglobin-Derivates entnommen, um die Plasma-Hämoglobin-Konzentration zum Zeitpunkt der Injektion (Zeitpunkt $t_0$) zu bestimmen, also die Konzentration des Hämoglobin-Derivates, die theoretisch bei Zugabe und gleichzeitiger vollständiger Vermischung vorliegen würde. Dafür können der zu untersuchenden Person beispielsweise zwei bis drei Blutproben entnommen werden, um über die Extrapolierung der Regressionsgerade die Konzentration zum Zeitpunkt $t_0$ zu ermitteln.

Aus diesen Werten kann das Plasmavolumen des Patienten berechnet werden. Gemäß einer bevorzugten Ausführungsform der Erfindung wird das Plasmavolumen nach der Formel

$$PV = D/C_{0Plasma}$$

berechnet, worin

PV das Plasmavolumen (in ml),

D die verabreichte Dosis des Hämoglobin-Derivates (in mg) und

$C_{0Plasma}$ die Konzentration des Hämoglobin im Plasma zum Injektionszeitpunkt darstellt, wobei sich $C_{0Plasma}$ aus der mittels Regressionsgerade bestimmten Plasma-Hämoglobin-Konzentration des Derivates abzüglich der Konzentration des Plasma-Hämoglobins vor Injektion des Derivates (des "Null-Wertes") ergibt.

Aus dem Plasmavolumen kann gemäß einer weiteren Ausführungsform der Erfindung das Blutvolumen nach folgender Formel berechnet werden:

$$BV = PV \cdot 100 / (100 - \text{Hematokrit}),$$

worin BV das Blutvolumen bezeichnet.

Gemäß einer alternativen Ausführungsform der Erfindung wird das Plasma- und/oder Blutvolumen über die Konzentration des von den Hämoglobin-Derivaten gebundenen Liganden bestimmt.

Selbstverständlich kann auch die Messung der Konzentration des Hämoglobin-Derivates und der Konzentration des davon gebundenen Liganden an einer Probe durchgeführt werden, wobei die Analyseverfahren voneinander verschieden sind.

Bei der Bestimmung der Konzentration des von den Hämoglobin-Derivaten gebundenen Liganden ist es erfindungsgemäß besonders bevorzugt Hämoglobin-Derivate zu verwenden, die mindestens teilweise Kohlenmonoxid als Liganden gebunden haben.

Der Kohlenmonoxid-Gehalt im Blut des Menschen beträgt normalerweise 0 bis 0,8 Vol.-% bezogen auf den Kohlenmonoxid-Gehalt in der Atemluft (Lehrbuch der Klinischen Chemie und Pathobiochemie, 3. Auflage, Tab. 6.4-.6), wobei in Ausnahmefällen bis zu 2,3% gemessen wurden. Bei Rauchern beträgt der Wert bis zu 5%. Die Zugabe von an Hämoglobin-Derivate gebundenem Kohlenmonoxid ist somit für die Patienten ungefährlich, da Kohlenmonoxid natürlicherweise im Blut vorkommt und die Sauerstoff-Transportkapazität des Eigenblutes des Patienten nicht beeinträchtigt wird.

Die Bestimmung des Plasma- und/oder Blutvolumens kann bei dieser Ausführungsform über die Bestimmung des Kohlenmonoxid-Gehaltes des Blutes erfolgen. Dieses Vorgehen hat den besonderen Vorteil, daß die Bestimmung des Kohlenmonoxid-Gehaltes im Vollblut mit besonders kleinen Probenmengen und in kürzester Zeit durchgeführt werden kann. Ein weiterer Vorteil dieses Vorgehens ist die hohe Genauigkeit der Bestimmung.

Auch bei dieser Ausführungsform der Erfindung kann es vorteilhaft sein, mindestens eine Probe von dem Patienten vor der Zugabe des an Hämoglobin-Derivate gebundenem Kohlenmonoxid zu entnehmen, um die Konzentration des Kohlenmonoxides im Blut des Patienten vor der Zugabe von weiterem Kohlenmonoxid zu bestimmen (Ermittlung des "Null-Wertes").

Die Applikation des Hämoglobin-Derivates kann, wie bereits oben beschrieben, durch zentral oder peripher venöse Injektion erfolgen. Beispielsweise können dem Patienten 20 bis 100 ml einer 5 bis 20 Gew.-/Vol.-%igen Lösung der Hämoglobin-Derivate in 2 bis 15 min injiziert werden, wodurch ein Kohlenmonoxid-Anstieg im Blut von etwa 2,8% erzeugt wird. Im Einzelfall hängt der Anstieg der Kohlenmonoxid-Konzentration im Blut von dem Gesamt-Hämoglobingehalt des Patienten ab.

Die Entnahme der Proben erfolgt, wie oben beschrieben. Auch bei diesem Verfahren kann es vorteilhaft sein, mehrere Proben zu entnehmen, um über die Extrapolierung einer Regressionsgerade die Konzentration des Kohlenmonoxid im Blut zum Injektionszeitpunkt zu ermitteln.

Zur Bestimmung des Kohlenmonoxid-Gehaltes im Blut ist die Verwendung von Blutgasanalysegeräten oder CO-Oxymetrie mit einem optischen Meßprinzip im Rahmen der vorliegenden Erfindung bevorzugt. Dabei sind Messungen bei 535, 560 und/oder 577 nm besonders bevorzugt. Das Volumen der für diese Messungen benötigten Probe beträgt vorzugsweise nicht mehr als 35 µl Probe. Die Analyse ist zudem sehr schnell und kann in 2,5 Minuten durchgeführt werden.

Die Plasma-Hämoglobin-Konzentration kann nach der Messung aus folgender Gleichung ermittelt werden:

$$nHb = nFCO\text{-}HB/dCO\text{-}Hb,$$

worin

nHb        die gesamte zirkulierende Menge an Hämoglobin in mmol,

nFCO-Hb   die verabreichte Menge an Kohlenmonoxid in mmol und

dCO-Hb    den Anstieg der Konzentration des Kohlenmonoxides im Blut nach der Kohlenmonoxid-Gabe bezeichnet (entspricht der Differenz aus der Messung vor der Gabe des Kohlenmonoxides und der Messung danach).

Gemäß einer weiteren Ausführungsform der Erfindung kann daraus das Blutvolumen nach der Formel

$$BV = nHb/cHb$$

berechnet werden, worin cHb die Hämoglobin-Konzentration in mmol/l bezeichnet und die anderen Abkürzungen die oben genannten Bedeutungen haben.

Die vorliegende Erfindung betrifft ferner ein Mittel zur Bestimmung des Plasma- und/oder Blutvolumens, das Hämoglobin-Derivate enthält, die mindestens teilweise Kohlenmonoxid als Liganden gebunden haben.

Auch für diese Ausführungsform der Erfindung ist es besonders bevorzugt, daß die Hämoglobin-Derivate ein Molekulargewicht von mindestens 128 kDa aufweisen.

Das Mittel zur Diagnose des Blutvolumens kann zusätzlich pharmazeutisch akzeptable Träger-, Hilfsstoffe und Reduktionsmittel enthalten, wobei die oben genannten Verbindungen bevorzugt sind.

Die besonderen Vorteile der erfindungsgemäßen Verwendung der Hämoglobin-Derivate liegen somit insbesondere darin, daß das Plasma- und/oder Blutvolumen eines Patienten unaufwendig und sehr schnell im klinischen Alltag bestimmt werden kann. Wiederholte Messungen sind möglich. Zur Ermittlung der Meßwerte aus den Proben können die im Stand der Technik üblichen Analysegeräte (Spektrophotometer, Densitometer, Blutgasanalysegerät/Oximetrie) verwendet werden, die eine automatische Aufarbeitung und Analyse der Proben ermöglichen. Die Ermittlung des Plasma- und/oder Blutvolumens aus den Meßwerten kann mittels eines Computers durchgeführt werden, so daß eine vollautomatische Auswertung der Proben gewährleistet ist und der Arzt lediglich den gemessenen Wert des Plasma- und/oder Blutvolumens ablesen muß.

**Patentansprüche**

1. Verwendung von Hämoglobin-Derivaten zur Bestimmung des Plasma- und/oder Blutvolumens, wobei es sich bei den Hämoglobin-Derivaten um stabile Chromoproteine mit einem Molekulargewicht von mindestens 64 kDa handelt.

2. Verwendung nach Anspruch 1, dadurch gekennzeichnet, daß die Hämoglobin-Derivate $\alpha$- und/oder $\beta$-Globinketten oder deren allelischen Varianten enthalten.

3. Verwendung nach Anspruch 1 oder 2, dadurch gekennzeichnet, daß die Hämoglobin-Derivate ein Molekulargewicht von mindestens 128 kDa aufweisen.

4. Verwendung nach einem der Ansprüche 1 bis 3, dadurch gekennzeichnet, daß die Hämoglobin-Derivate zusammen mit Träger- und/oder Hilfsstoffen verwendet werden.

5. Verwendung nach Anspruch 4, dadurch gekennzeichnet, daß als Träger- und/oder Hilfsstoffe physiologische Lösungen, wie Ringer Laktat, Natriumchlorid, Natriumphosphat, Polysorbate und/oder EDTA verwendet werden.

6. Verwendung nach einem der Ansprüche 1 bis 5, dadurch gekennzeichnet, daß die Hämoglobin-Derivate zusammen einem Reduktionsmittel oder Stabilisator verwendet werden.

7. Verwendung nach Anspruch 6, dadurch gekennzeichnet, daß Ascorbinsäure, N-Acetyltryptophan oder N-Acetylcystein als Stabilisator verwendet wird.

8. Verwendung nach einem der Ansprüche 1 bis 7, dadurch gekennzeichnet, daß die Hämoglobin-Derivate in einem Verfahren verwendet werden, bei dem man

   (a) einem Patienten eine bestimmte Menge des Hämoglobin-Derivates appliziert,
   (b) die Vermischung des Hämoglobin-Derivates mit dem Blut des Patienten abwartet,

   (c) dem Patienten mindestens eine Probe entnimmt,
   (d) den Gehalt des Hämoglobin-Derivates und/oder den Gehalt des von den Hämoglobin-Derivaten gebundenen Liganden in der Probe bestimmt und
   (e) das Plasma- und/oder Blutvolumen daraus ermittelt.

9. Verwendung nach einem der Ansprüche 1 bis 8, dadurch gekennzeichnet, daß die Hämoglobin-Derivate mindestens teilweise Kohlenmonoxid als Liganden enthalten und der Kohlenmonoxid-Gehalt in der Probe des Patienten bestimmt wird.

10. Mittel zur Bestimmung des Plasma- und/oder Blutvolumens, das Hämoglobin-Derivate enthält, dadurch gekannzeichnet, daß es sich bei den Hämoglobin-Derivaten um stabile Chromoproteine mit einem Molekulargewicht von mindestens 64 kDa handelt, die mindestens teilweise Kohlenmonoxid als Liganden tragen.

11. Mittel nach Anspruch 10, dadurch gekennzeichnet, daß die Hämoglobin-Derivate ein Molekulargewicht von mindestens 128 kDa aufweisen.

12. Mittel nach Anspruch 10 oder 11, dadurch gekennzeichnet, daß das Mittel zusätzlich pharmazeutisch akzeptable Träger- und/oder Hilfsstoffe enthält.

13. Mittel nach Anspruch 12, dadurch gekennzeichnet, daß als Träger- und/oder Hilfsstoffe physiologische Lösungen, wie Ringer Laktat, Natriumchlorid, Natriumphosphat, Polysorbate und/oder EDTA verwendet werden.

14. Mittel nach einem der Ansprüche 10 bis 13, dadurch gekennzeichnet, daß das ferner Mittel einen Stabilisator enthält.

15. Mittel nach Anspruch 14, dadurch gekennzeichnet, daß Ascorbinsäure, N-Acetyltryptophan oder N-Acetylcystein als Stabilisator verwendet wird.

16. Verfahren zur Herstellung eines Mittels nach einem der Ansprüche 10 bis 15, bei dein man Hämoglobin-Derivate mit einer physiologischen Lösung vermischt, dadurch gekennzeichnet, daß es sich bei den Hämoglobin-Derivaten um stabile Chromoproteine mit einem Molekulargewicht von mindestens 64 kDa handelt, die mindestens teilweise Kohlenmonoxid als Liganden tragen.

17. Verfahren nach Anspruch 16, bei dem man zusätzlich geeignete Hilfs- oder Trägerstoffe zugibt.

18. Verfahren nach Anspruch 17, wobei Ringer Laktat, Natriumchlorid, Natriumphosphat, Polysorbate und/oder EDTA enthaltende Lösungen als Hilfs- oder Trägerstoffe verwendet werden.

19. Verfahren nach einem der Ansprüche 16 bis 18, bei dem man zusätzlich geeignete Stabilisatoren zugibt.

20. Verfahren nach Anspruch 19, wobei Ascorbinsäure, N-Acetyltryptophan oder N-Acetylcystein als Stabilisator verwendet werden.